# EUROPEAN PATENT SPECIFICATION

(11) **EP 1 299 350 B1**
(45) Date of publication and mention of the grant of the patent: **29.09.2004**
(21) Application number: 01958717.9
(22) Date of filing: 03.07.2001
(51) Int. Cl.: C07C 233/78, C07D 295/04, A61K 31/166, A61K 31/167, A61K 31/495, A61K 31/5375, A61P 37/04, A61P 31/00, A61P 25/18, A61P 25/24, A61P 35/00

(54) **SUBSTITUTED BENZAMIDES FOR IMMUNE ENHANCEMENT AND FOR THE TREATMENT OF CANCER, INFECTION AND MANIC-DEPRESSIVE ILLNESS**
SUBSTITUIERTE BENZAMIDE FÜR DIE IMMUNSTÄRKUNG, DIE BEHANDLUNG VON KREBS, INFEKTIONEN UND MANISCH-DEPRESSIVE ERKRANKUNGEN
BENZAMIDES SUBSTITUES PERMETTANT D'AUGMENTER L'EFFET IMMUNITAIRE ET DE TRAITER LE CANCER, LES INFECTIONS ET LA MALADIE AFFECTIVE BIPOLAIRE

(30) Priority: 05.07.2000 SE 0002531
(43) Date of publication of application: 09.04.2003
(73) Proprietor: Active Biotech AB, 220 07 Lund (SE)
(72) Inventor: BJÖRK, Anders, S-230 50 Bjärred (SE); HEDLUND, Gunnar, S-224 56 Lund (SE); LEANDERSON, Tomas, S-211 16 Malmö (SE)
(74) Representative: Bergvall-Eftring, Stina Lena
(86) International application number: PCT/SE2001/001526
(87) International publication number: WO 2002/002511

(56) References cited:
- BE-A- 620 543
- ERIC D. PESELOW ET AL.: 'Clinical trials of benzamides in psychiatry' ADVANCED IN BIOCHEMICAL PSYCHOPHARMACOLOGY vol. 35, 1982, pages 163 - 194, XP002948158
- RONALD W. PERO ET AL.: 'Newly discovered anti-inflammatory properties of the benzamides and nicotinamides' MOLECULAR AND CELLULAR BIOCHEMISTRY vol. 193, 1999, pages 119 - 125, XP002948157

## Description

### FIELD OF THE INVENTION

The present invention relates to substituted benzamides, which are enhancers of transcription factor AP (activator protein) -1, to compositions containing them, and to methods for clinical treatment of diseases associated with immune suppressive states and to the use of the benzamides for the preparation of a medicament for stimulation of transcription factor AP-1. Such compounds are particularly useful in the treatment of a variety of diseases associated with immune suppression and low capability to produce IL (interleukin) -2. Such diseases include cancer, autoimmune disease and infectious disease. More particularly, the present invention relates to benzamide derivatives suitable for the treatment of, for example, solid tumours, rheumatoid arthritis (RA) and AIDS. The compounds of the present invention are also suitable for the treatment of manic-depressive illness.

### BACKGROUND OF THE INVENTION

AP-1 is a transcriptionally active protein heterodimer containing members of the Fos (e.g. c-Fos, FosB, Fra-1, Fra-2) and the Jun (e.g. c-Jun, JunB, JunD) family of proteins. The AP-1 transcription factors are stimulated by e.g. growth factors, cytokines, T cell activators and neurotransmitters and act as dimers binding to DNA in many promoters including proteases and cytokines like IL-2. C-Jun and c-Fos knock-out mice have been produced showing embryonic lethality and osteopetrosis / lymphopenia / behavioural abnormalities, respectively (Johnson et al., 1992). These results emphasise that the AP-1 site is pivotal in many different genes and points out lymphocyte regulation and behaviour, regulation in the central nervous system, as two distinct biological hotspots. Therefore, immune suppression with low capability to produce IL-2 and behavioural disorders are two very different medical indication areas where AP-1 activity is suboptimal and where AP-1 enhancers can be applied.

In US Patent No. 3,177,252 some substituted benzamide derivatives including metoclopramide (The Merck Index 12^{th} Ed., entry 6226) are disclosed as being useful for the treatment of emesis.

The compound metoclopramide may be associated with depression and, because of its central as well as peripheral dopamine-blocking properties, may cause most unwanted tardive dyskinesia. Structure-activity relationship studies have demonstrated the link between the diaminoethylene bridge and the dopamine-D₂ blockade.

In GB 1,174,956 quaternary ammonium salts of N-substituted benzamide derivatives and their action to accelerate the automatic motility of digestive tract are disclosed.

In J. Org. Chem. USSR 22, 578-582 (1986), the synthesis ofN-(3-dimethylamino-propyl)-3-nitro-4-acetylaminobenzamide is described.

In US Patent No. 4,568,685 some N-[(1H-1,2,4-triazol-1-yl)alkyl]arylamides are disclosed as being inhibitors of thromboxane synthetase enzyme.

In US Patent No. 4,568,687 some N-[ω(1H-imidazole-1-yl)alkyl]arylamides are disclosed as being inhibitors of thromboxane synthetase enzyme and are also useful in the treatment of hypertension and myocardial ischemia.

In Analytical Profiles of Drug Substances vol. 4, K Florey, Ed. (Academic Press, New York, 1975) pp 333-383, procainamide (The Merck Index 12^{th} Ed., entry 7936) is described as an antiarrhytmic agent.

We have now discovered a novel method of stimulating the transcription factor AP-1 using substituted benzamides.

### SUMMARY OF THE INVENTION

### Immune suppression in cancer, autoimmune disease and infectious disease

Immune system-based approaches for the treatment of malignant disease have focused on cytolytic effector cells such as cytotoxic T lymphocytes (CTL), and natural killer (NK) cells. It has also been demonstrated that tumour-bearing mice can be cured using a wide variety of approaches, some of which involve IL-2 mediated enhancement of CTL and NK cell activity.

However, the apparent success in mice stands in contrast to the current situation in the clinic, wherein only a minority of patients have thus far benefited from CTL- or NK cell-based antitumour approaches. This is probably a result from tumour-induced immune suppression (Whiteside, 1999; Kiessling et al., 1999). One of the underlying causes of tumour-associated immune suppression of CTL and NK cell activity, the intracellular signalling deficiency resulting from reduction of the TCR/CD3 zeta chain expression of the T cells, is also shared with HIV infection, leprosy, and rheumatoid arthritis. This signalling deficiency is overrun by IL-2 treatment in vitro. IL-2 is a central cytokine in the development of functional immune responses and it has been clearly shown that the AP-1 site of the IL-2 promoter is pivotal for optimal activity (Sundstedt and Dohlsten, 1998). Distinct benzamides would therefore represent an alternative treatment for immune stimulation and IL-2 enhancement in immune suppressive states of disease. In addition, several treatment regiments such as cytostatic and radiation therapy applied in the treatment of cancer result in unwanted immune suppression, an induced state that would be compensated for by administering compounds of the present invention.

### Manic-depressive illness

Lithium and sodium valproate (VPA) (The Merck Index 12^{th} Ed., entry 10049) are effective in the treatment of bipolar disorders (manic-depressive illness) and may function through the regulation of signal transduction pathways and transcription factors such as c-Fos and c-Jun, which in turn results to changes in gene expression. The long-term efficacy of lithium and VPA in bipolar disorders suggests that the regulation of gene expression may be an important target for these drugs. These two structurally highly dissimilar agents, lithium and VPA, increase AP-1 DNA binding activity in areas of rodent brain ex vivo and in human neuronal cells in culture (Yuan et al., 1998; Chen et al., 1999). Both treatments also increase the expression of a reporter gene driven by an AP-1-containing promoter, and mutations in the AP-1 sites of the reporter gene promoter markedly attenuate these effects. Both treatments also increase the expression of several endogenous proteins, which genes are known to be regulated by AP-1. These effects suggest that the temporal regulation of AP-1 mediated gene expression in critical neuronal circuits may play a role in the long-term therapeutic efficacy of lithium and VPA and point out that also other AP-1 enhancers like distinct benzamides could potentially act as modulators of e.g. manic-depressive illness.

### DESCRIPTION OF THE INVENTION

A primary objective of the present invention is to provide benzamide compounds which by virtue of their pharmacological profile, with high potency in experimental models and low level of side-effects, are considered to be of value in the treatment of disease associated with immune suppressive states e.g. for the stimulation, enhancement or modulation of the immune response. Included in the invention is also the use of the compounds for the preparation of a medicament for the stimulation of transcription factor AP-1. In a particular aspect, this invention provides preparation of a medicament for the stimulation of transcription factor AP-1, a method of treating diseases in which the disease pathology may be therapeutically modified by stimulating AP-1. Examples of such diseases are cancer, autoimmune disease and infectious disease. More particularly, the present invention relates to benzamide derivatives suitable for the treatment of, for example, solid tumours, rheumatoid arthritis (RA) and AIDS. The compounds of the present invention are also suitable for the treatment of manic-depressive illness.

The term "treatment" as used herein includes prophylaxis as well as relieving the symptoms of disease.

It has now surprisingly been found that the compounds of general formula (I) 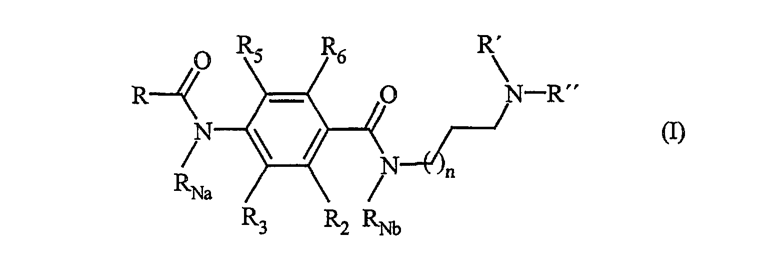 wherein
R is selected from methyl, ethyl, *n*-propyl, *iso*-propyl, *c*-propyl, *n*-butyl, *sec*.-butyl, *iso*-butyl, *tert*.-butyl, *c*-butyl, *n*-pentyl, *sec*.-pentyl, *iso*-pentyl, *tert*.-pentyl, *neo*-pentyl, *c*-pentyl, *c*-hexyl and *c*-heptyl;
R_{Na} and R_{Nb} are the same or different and selected from hydrogen, methyl and ethyl;
R₂, R₃, R₅ and R₆ are independently selected from hydrogen, methyl, methoxy, thiomethyl, hydroxy, fluoro, chloro, bromo, trifluoromethyl, phenyl and benzyl;
n is 1, 2 or 3;
R' and R" are the same or different and selected from methyl, ethyl, *n*-propyl, *iso*-propyl, *n* -butyl, *sec*.-butyl, and *iso*-butyl or R' and R" together form a saturated heterocyclic ring of 5-7 atoms having the formula 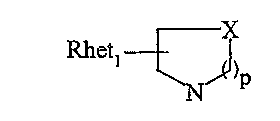 wherein p is 1, 2, 3;
X is selected from CHRhet₁, NRhet₁ and O with the proviso that p is 2 or 3 when X is NRhet₁ and O;
Rhet₁ is selected from hydrogen and C₁₋₅ alkyl, optionally functionalised with OH, halogen (F, Cl and Br), CN, COORhet₂, N(Rhet₂)₂ wherein Rhet₂ independently is selected from H, C₁-₄ alkyl;
with the proviso that when R' and R'' are methyl then R cannot be methyl;
and pharmaceutically acceptable salts, hydrates and solvates thereof;
are unexpectedly effective and specific in the treatment of individuals suffering from cancer, autoimmune disease, infectious disease and manic-depressive illness.

In a preferred embodiment of the invention
R is selected from methyl, ethyl, *n*-propyl and *iso*-propyl,
R_{Na} and R_{Nb} are hydrogen,
one of R₂, R₃, R₅ and R₆ is selected from methyl, methoxy, thiomethyl, hydroxy, fluoro, chloro, trifluoromethyl and phenyl,
R' and R" are selected from methyl, ethyl, *n*-propyl and *n*-butyl
with the proviso that when R' and R" are methyl then R cannot be methyl;.

The compounds of general formula (I) were assayed for AP-1 enhancement. The compounds of this invention were tested in assays for the modulation of stimulated activity of an AP-1-driven reporter gene in the Jurkat T cell line. The activation of this reporter gene results in luciferase production and the amounts of produced luciferase parallels the level of AP-1 activity. A high level of AP-1 activity is pivotal to e.g. high production of IL-2.

All embodiments of the invention as disclosed in the claims are herewith included in the specification.

The following examples are intended to illustrate the invention without restricting the scope thereof.

The compounds of general formula (I) may be prepared by methods well known in the literature. The general solution preparation is shown in Scheme 1 and 2.

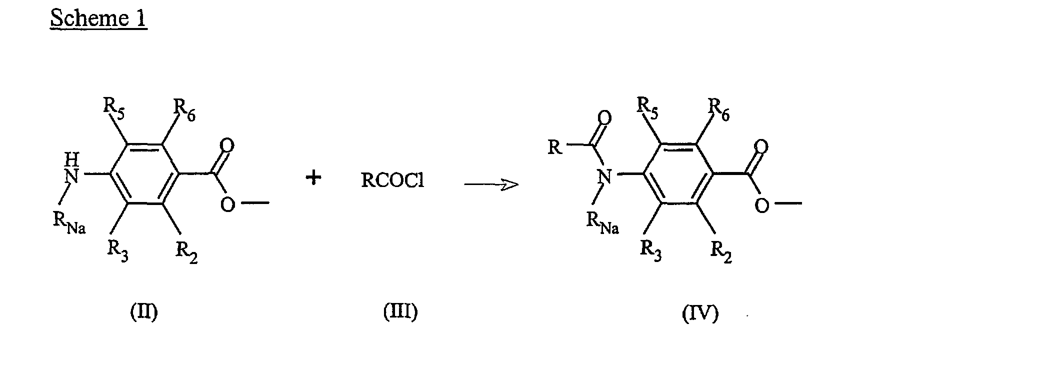

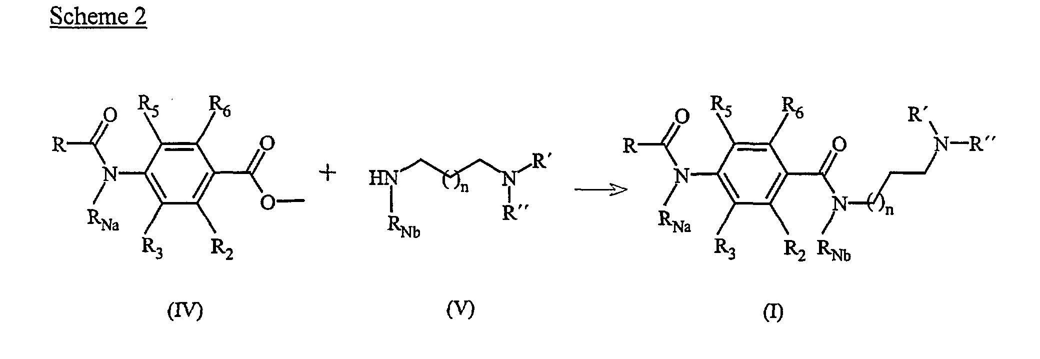

A benzamide derivative of general formula (I) may be prepared by conventional methods and, for example, by first the reaction of a 4-amino-benzoic acid methyl ester (II) with an acid chloride (III) or anhydride in an inert solvent such as dichloromethane in the presence of triethylamine (Scheme 1). The resulting 4-acylamino-benzoic acid methyl esters (IV) and a N,N-substituted alkylenediamine (V) is then condensed in an excess of V in the presence of a catalytic amount ammonium chloride to form the compounds of general formula (I) (Scheme 2). Alternatively, the methylester is first hydrolysed and then activated using conventional methods, such as ethylchloroformate, dicyclohexylcarbodiimide (DCC) or 2-(1H-benzotriazol-1-yl)-1,1,3,3-tetramethyluronium tetrafluoroborat (TBTU). The starting materials used herein are commercially available or are prepared by conventional methods found in standard reference books such as the Compendium of Organic Synthetic Methods, vol. I-VI (Wiley-Interscience), well known to those of ordinary skill in the art.

Acid addition salts of the compounds of formula (I) are prepared in a standard manner in a suitable solvent and in excess of an acid, such as hydrochloric, hydrobromic, sulphuric, maleic and succinic acid.

### Example 1

### 4-iso-Butyrylamino-2-methoxy-benzoic acid methyl ester.

1.81 g of 4-amino-2-methoxy-benzoic acid methyl ester and 1.4 g of triethylamine were dissolved in 20 ml of dichloromethane. 1.23 g of isobutyryl chloride in 5 ml of dichloromethane was added dropwise at 0°C. The reaction mixture was allowed to reach room temperature and stirred for 3 hours. The reaction mixture was washed with water, dried and the solvents were evaporated to yield 1.9 g of the title product.
1H NMR (CDCl₃): δ 1.22 (6H, d), 2.53 (1H, m), 3.83 (3H, s), 3.83 (3H, s), 6.85 (1H, d), 7.70 (1H, s), 7.75 (1H, d), 7.84 (1H, s).

### Example 2

### N-[(3-Diethylamino)propyl]-4-isobutyrylamino-2-methoxy-benzamide.

0.3 g of 4-iso-butyrylamino-2-methoxy-benzoic acid methyl ester was dissolved in 3 ml of N,N-diethyl-propylenediamine together with a catalytic amount of ammonium chloride. The reaction mixture was refluxed for 3 hours. Dichloromethane was added and washing 4 times with water removed excess diamine. Drying and evaporation of the solvents yielded 0.15 g of the title compound.
1H NMR (CDCl₃): δ 0.97 (6H, t), 1.20 (6H, d), 1.71 (2H, m), 2.48 (6H, m), 2.59 (1H, m), 2.46 (2H, q), 3.90 (3H, s), 6.80 (1H, d), 7.94 (1H, s), 8.03 (1H, d), 8.29 (1H, s).

### Example 3

### N-[3-(Diethylamino)propyl]-4-iso-buturylamino-3-hydroxy-benzamide

4-iso-Butyrylamino-3-hydroxy-benzoic acid (0.089 g) and TBTU (0.128 g) in chloroform (15 ml) was stirred for 2 hours. N,N-Diethyl-propylenediamine (0.052 g) was added and the mixture was stirred for 1 hour. Evaporation of the solvent gave a residue, which was chromatographed on a silica gel column first eluted/washed with ethylacetate and then eluted with ethylacetate-methanol-triethylamine (12:4:1) to yield 0.03 g, of the title product.
1H NMR (CDCl₃): δ 1.14 (t, 6H) 1.25 (m, 6H) 2.01 (m, 2H) 2.61 (m, 1H) 2.98 (m, 2H) 3.04 (m, 2H) 3.44 (m, 2H) 7.35 (m, 1H) 7.80 (d, 1H) 7.92 (m, 1H) 8.21 (d, 1H) 8.36 (s, 1H).

### Example 4

### 4-(N-iso-Butyryl-N-methylamino)-benzoic acid

4-Methylamino-benzoic acid methyl ester (0.41 g) were suspended in chloroform (20 ml). Isobutyryl chloride (0.79 g) in chloroform (10 ml) was added dropwise for 30 minutes and then dropwise triethylamine (1 ml) dissolved in chloroform (5 ml). The reaction mixture was stirred for 3 hours and the solvent was evaporated. 1M NaOH(aq) (40 ml) was added and the mixture stirred overnight. The solution was filtered to remove unsolved material and then acidified with 2M HCl(aq). Filtration and drying in vacuum yielded 0.48 g of the title product.

### Example 5

### N-[3-(Diethylamino)propyl]-4-(N-iso-butyryl-N-methylamino)-benzamide

To a stirred solution of 4-(iso-butyryl-N-methylamino)-benzoic acid (0.088 g) and triethylamine (0.041 g) in chloroform (4 ml) was added a solution of ethyl chlorformate (0.049 g) in chloroform (1 ml). The mixture was stirred under an N₂ atmosphere at room temperature for 1½ hours and then cooled to 0 °C. N,N-Diethyl-propylenediamine (0.051 g) in chloroform (1 ml) was added and the mixture was stirred over night. The mixture were washed with 0.5 M NaOH(aq) and water. Drying and evaporation of the solvent gave a residue, which was chromatographed on a silica gel column first eluted/washed with ethylacetate and then eluted with ethylacetate-methanol-triethylamine (12:4:1) to yield 0.098 g, of the title product.
1H NMR (CDCl₃): δ 1.01 (t, 6H) 1.04 (t, 6H) 1.77 (m, 2H) 2.48 (m, 1H) 2.61 (m, 6H) 3.23 (s, 3H) 3.57 (m, 2H) 7.21 (m, 1H) 7.83 (m, 1H) 8.80 (s, 1H).

Examples 6 to 12 were prepared by the method described in Example 5.

### Example 6

### N-[3-(Diethylamino)propyl]-4-iso-butyrylamino-3-methoxy-benzamide

1NMR (CDCl₃): δ 1.07 (t, 6H) 1.25 (d, 6H) 1.80 (m, 2H) 2.55 (m, 1H) 2.64 (m, 6H) 3.56 (m, 2H) 3.94 (s, 3H) 7.27 (m, 1H) 7.55 (d, 1H) 7.91 (s, 1H) 8.43 (d, 1H) 8.70 (s, 1H).

### Example 7

### N-[3-(Diethylamino)propyl]-4-(iso-butyryl-N-methylamino)-2-methoxy-benzamide

1H NMR (CDCl₃): δ 1.04 (m, 12H) 1.80 (m, 2H) 2.56 (m, 7H) 3.24 (s, 3H) 6.75 (s, 1H) 6.88 (m, 1H) 7.99 (m, 1H) 8.17 (d, 1H).

### Example 8

### N-(3-morpholinopropyl)-4-iso-butyrylamino-2-methoxy-benzamide

1H NMR (CDCl₃): δ 1.24 (d, 6H) 1.86 (m, 2H) 2.52 (m, 7H) 3.51 (m, 2H) 3.75 (m, 4H) 3.97 (s, 3H) 6.71 (m, 1H) 7.37 (s, 1H) 7.94 (s,1H) 8.09 (d, 1H).

### Example 9

### N-[4-(Dimethylamino)butyl]-4-iso-butyrylamino-2-methoxy-benzamide

1H NMR (CDCl₃): δ 1.25 (d, 6H) 1.65 (m, 4H) 2.37 (m, 6H) 2.52 (m, 3H) 3.45 (m, 2H) 3.97 (s, 3H) 6.73 (m, 1H) 7.43 (s, 1H) 7.88 (m, 1H) 7.92 (s, 1H) 8.09 (d, 1H).

### Example 10

### N-[3-(4-methylpiperazino)-propyl]-4-iso-butyrylamino-2-methoxy-benzamide

1H NMR (CDCl₃): δ 1.25 (d, 6H) 1.83 (m, 3H) 2.35 (s, 3H) 2.52 (m, 3H) 2.60 (m, 7H) 3.49 (m, 2H) 3.96 (s, 3H) 6.71 (m, 1H) 7.37 (s, 1H) 7.94 (m, 2H) 8.08 (d, 1H).

### Example 11

### N-[3-(Diethylamino)propyl]-4-iso-butyrylamino-3,5-dichloro-benzamide

1H NMR (CDCl₃): δ 1.10 (t, 6H) 1.29 (d, 6H) 1.82 (m, 2H) 2.69 (m, 7H) 3.55 (m, 2H) 7.03 (s, 1H) 7.82 (s, 2H) 9.22 (s, 1H).

### Pharmacological methods

Cells from the Jurkat T cell line were transfected with an AP-1-driven luciferase gene reporter construct (Parra et al., 1997) together with a selection gene vector. Selected clones from the resulting stable AP-1 reporter gene transfectants were used in the assays for AP-1 transcription factor activity. These transfected Jurkat cells (Jurkat/AP-1rep) were grown in RPMI 1640 supplemented with glutamine, hepes, sodium pyruvate, gentamicin, 10% FCS and G418. To evaluate distinct compounds for their capacity to enhance the AP-1 activity, Jurkat/AP-1rep cells were stimulated for 5.5 h in the temperature of 37°C with phorbol myristate acetate and ionomycin in the absence and presence of test compounds. The 96 well plates containing the cell cultures were then put on ice until harvested. The supernatants were removed and the cells were lysed. AP-1 activity was measured as luminiscence produced by luciferase substrate, which was added to the wells in conjunction with measurement.

Superantigen responsive mice were treated with Staphylococcal enterotoxin A in accordance with Belfrage et al. (Belfrage et al. 1995, 1997a, 1997b). Plasma and splenocytes were collected at different time points to evaluate the induced activity ofT cells with and without treatment with the compounds of general formula (I). It is shown that T cell activity as measured as IL-2 production, cytotoxic T cell activity and anergy induction (Belfrage et al. 1995, 1997a, 1997b) were modulated by the treatment.

Among preferred compounds is N-[(3-diethylamino)propyl]-4-isobutyrylamino-2-methoxybenzamide, hereinafter called Compound A. Compound A was shown to enhance the activity of the AP-1 driven reporter in a dose dependent fashion down to µM concentrations (Figure 1).

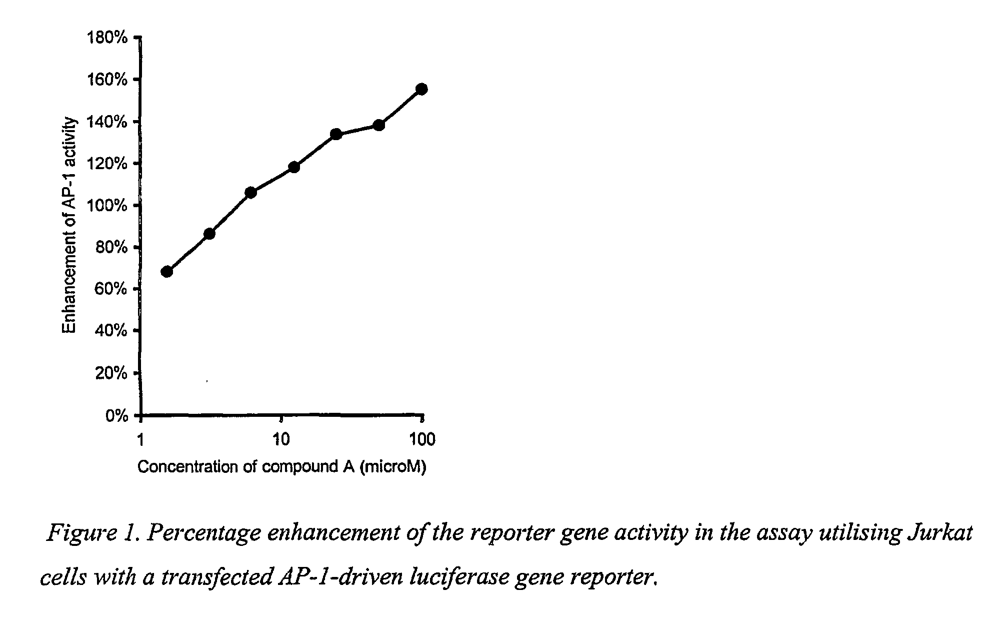

The compounds of formula (I) are useful as enhancers of AP-1. The present invention provides useful compositions and formulations of said compounds including pharmaceutical compositions and formulations of said compounds.

Effective quantities of the compounds of formula (I) are preferably administered to a patient in need of such treatment according to usual routes of administration and formulated in usual pharmaceutical compositions comprising an effective amount of the active ingredient and a suitable pharmaceutically acceptable carrier. Such compositions may take a variety of forms, e.g. solutions, suspensions, emulsions, tablets, capsules, and powders prepared for oral administration, aerosols for inhalations, sterile solutions for parental administration, suppositories for rectal administration or suitable topical formulations. Conventional procedures for the selection and preparation of suitable pharmaceutical formulations are described, for example, in "Pharmaceuticals - The Science of Dosage Form Design", M.B. Aulton, Churchill Livingstone, 1988.

A suitable daily dose for use in the treatment of disease with associated immune suppression or manic-depressive illness is contemplated to vary between 0.0005 mg/kg to about 10 mg/kg body weight, in particular between 0.005 mg/kg to 1 mg/kg body weight, depending upon the specific condition to be treated, the age and weight of the specific patient, and the specific patient's response to the medication. The exact individual dosage, as well as the daily dosage, will be determined according to standard medical principles under the direction of a physician.

Various additives to enhance the stability or ease of administration of the drug are contemplated. The pharmaceutical composition may also contain additional therapeutically useful substances other than a compound of formula (I).

The ability of the compounds of the present invention to enhance the AP-1 activity is clearly evidenced by their ability to enhance the reporter gene activity (see Figure 1). No unacceptable toxicological effects are expected such as tardive dyskinesia when compounds of the present invention are administered in accordance with the present invention.

### References

Belfrage H, Dohlsten M, Hedlund G, Kalland T. 1995 Enhanced and prolonged efficacy of superantigen-induced cytotoxic T lymphocyte activity by interleukin-2 in vivo. Cancer Immunol Immunother 1995 Aug; 41 (2):87-94

Belfrage H, Dohlsten M, Hedlund G, Kalland T. 1997a Prevention of superantigen-induced down-regulation of T-cell mediated cytotoxic activity by IL-2 in vivo. Immunology 1997 Feb; 90(2):183-8

Belfrage H, Dohlsten M, Hedlund G, Kalland T. 1997b Prevention of superantigen-induced tolerance in vivo by interleukin-2 treatment. Cancer Immunol Immunother 1997 Apr; 44(2): 77-82

Chen G, Yuan PX, Jiang YM, Huang LD, Manji HK. Valproate robustly enhances AP-1 mediated gene expression. Brain Res Mol Brain Res 1999 Jan 22; 64(1):52-8 Johnson RS, Spiegelman BM, Papaioannou V. Pleiotropic effects of a null mutation in the c-fos proto-oncogene. Cell 1992 Nov 13;71(4):577-86

Kiessling R, Wasserman K, Horiguchi S, Kono K, Sjoberg J, Pisa P, Petersson M. Tumor-induced immune dysfunction. Cancer Immunol Immunother 1999 Oct; 48(7):353-62

Parra E, Varga M, Hedlund G, Kalland T, Dohlsten M . Costimulation by B7-1 and LFA-3 targets distinct nuclear factors that bind to the interleukin-2 promoter: B7-1 negatively regulates LFA-3-induced NF-AT DNA binding. Mol Cell Biol 1997 Mar; 17(3): 1314-23

Sundstedt A, Dohlsten M. In vivo anergized CD4+ T cells have defective expression and function of the activating protein-1 transcription factor. J Immunol 1998 Dec 1;161(11): 5930-6

Whiteside TL. Signaling defects in T lymphocytes of patients with malignancy. Cancer Immunol Immunother 1999 Oct; 48(7):346-52

Yuan PX, Chen G, Huang LD, Manji HK. Lithium stimulates gene expression through the AP-1 transcription factor pathway. Brain Res Mol Brain Res 1998 Jul 15; 58(1-2):225-30

## Claims

1. Use of a compound of the general formula (I) 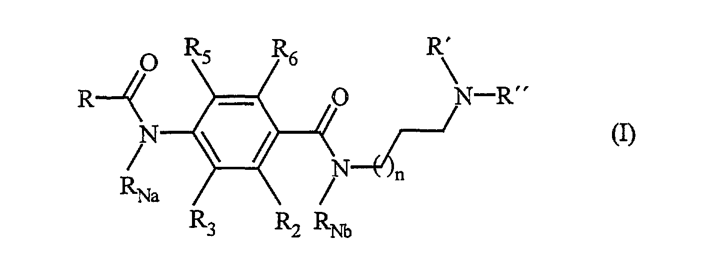 wherein
R is selected from methyl, ethyl, *n*-propyl, *iso*-propyl, *c*-propyl, *n*-butyl, *sec*.-butyl, *iso*-butyl, *tert*.-butyl, *c*-butyl, *n*-pentyl, *sec*.-pentyl, *iso*-pentyl, *tert*.-pentyl, *neo*-pentyl, *c*-pentyl, *c*-hexyl and *c*-heptyl;
R_{Na} and R_{Nb} are the same or different and selected from hydrogen, methyl and ethyl;
R₂, R₃, R₅ and R₆ are independently selected from hydrogen, methyl, methoxy, thiomethyl, hydroxy, fluoro, chloro, bromo, trifluoromethyl, phenyl and benzyl;
n is 1, 2 or 3;
R' and R" are the same or different and selected from methyl, ethyl, *n*-propyl, *iso*-propyl, *n*-butyl, *sec*.-butyl, and *iso*-butyl or R' and R" together form a saturated heterocyclic ring of 5-7 atoms having the formula 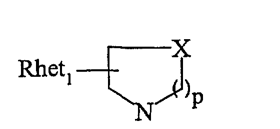 wherein p is 1, 2, 3;
X is selected from CHRhet₁, NRhet₁ and O with the proviso that p is 2 or 3 when X is NRhet₁ and O;
Rhet₁ is selected from hydrogen and C₁₋₅ alkyl, optionally functionalised with OH, halogen (F, Cl and Br), CN, COORhet₂, N(Rhet₂)₂ wherein Rhet₂ independently is selected from H, C₁-₄ alkyl; and pharmaceutically acceptable salts, hydrates and solvates thereof; for the preparation of a medicament for stimulation of transcription factor AP (activator protein)-1.

2. Use according to claim 1 of compounds of the general formula (I) for the preparation of a medicament for the stimulation, enhancement or modulation of the immune response.

3. Use according to claim 2 of compounds of the general formula (I) for the preparation of a medicament for the treatment of cancer and unwanted immune suppression induced by e.g. cytostatic and radiation therapy.

4. Use according to claim 2 of compounds of the general formula (I) for the preparation of a medicament for the treatment of autoimmune disease.

5. Use according to claim 2 of compounds of the general formula (I) for the preparation of a medicament for the treatment of infectious disease.

6. Use according to claim 1 of compounds of the general formula (I) for the preparation of a medicament for the treatment of manic-depressive illness.

7. Use according to claim 1 of N-[(3-diethylamino)propyl]-4-isobutyrylamino-2-methoxy-benzamide.

8. Use according to claim 1 of the compound for the preparation of a medicament to be administered in a daily dosage of between 0.0005 mg/kg to about 10 mg/kg body weight, in particular between 0.005 to 1 mg/kg body weight.

9. Composition comprising as an active ingredient compounds of the general formula (I) 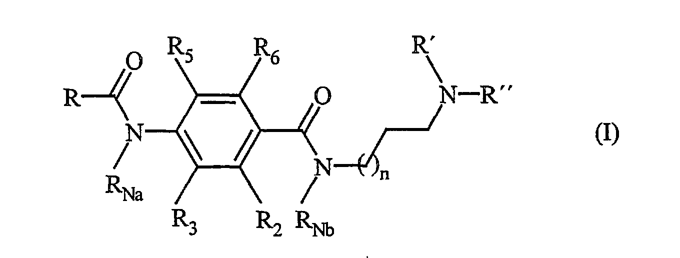 wherein
R is selected from methyl, ethyl, *n*-propyl, *iso*-propyl, *c*-propyl, *n*-butyl, *sec*.-butyl, *iso*-butyl, *tert*.-butyl, *c*-butyl, *n*-pentyl, *sec*.-pentyl, *iso*-pentyl, *tert*.-pentyl, *neo*-pentyl, *c*-pentyl, *c*-hexyl and *c*-heptyl;
R_{Na} and R_{Nb} are the same or different and selected from hydrogen, methyl and ethyl;
R₂, R₃, R₅ and R₆ are independently selected from hydrogen, methyl, methoxy, thiomethyl, hydroxy, fluoro, chloro, bromo, trifluoromethyl, phenyl and benzyl;
n is 1, 2 or 3;
R' and R" are the same or different and selected from methyl, ethyl, *n*-propyl, *iso*-propyl, *n*-butyl, *sec*.-butyl, and *iso*-butyl or R' and R" together form a saturated heterocyclic ring of 5-7 atoms having the formula 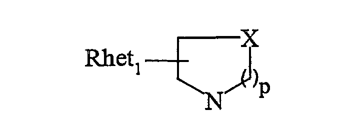 wherein p is 1, 2, 3;
X is selected from CHRhet₁, NRhet₁ and O with the proviso that p is 2 or 3 when X is NRhet₁ and O;
Rhet₁ is selected from hydrogen and C₁₋₅ alkyl, optionally functionalised with OH, halogen (F, Cl and Br), CN, COORhet₂, N(Rhet₂)₂, wherein Rhet₂ independently is selected from H, C₁-₄ alkyl; pharmaceutically acceptable salts, hydrates and solvates thereof and a pharmaceutically active carrier.

10. Compounds of general formula (I) 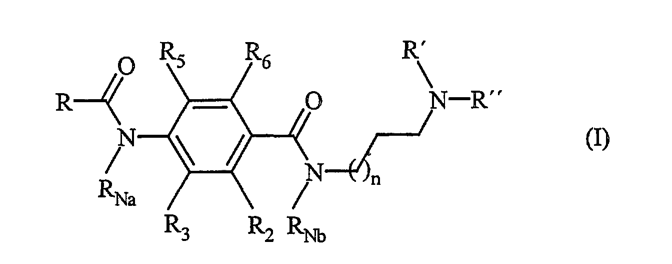 wherein
R is selected from methyl, ethyl, *n*-propyl, *iso*-propyl, *c*-propyl, *n*-butyl, *sec*.-butyl, *iso*-butyl, *tert*.-butyl, *c*-butyl, *n*-pentyl, *sec*.-pentyl, *iso*-pentyl, *tert*.-pentyl, *neo*-pentyl, *c*-pentyl, *c*-hexyl and *c*-heptyl;
R_{Na} and R_{Nb} are the same or different and selected from hydrogen, methyl and ethyl;
R₂, R₃, R₅ and R₆ are independently selected from hydrogen, methyl, methoxy, thiomethyl, hydroxy, fluoro, chloro, bromo, trifluoromethyl, phenyl and benzyl;
n is 1, 2 or 3;
R' and R" are the same or different and selected from methyl, ethyl, *n*-propyl, *iso*-propyl, *n* -butyl, *sec*.-butyl, and *iso*-butyl or R' and R" together form a saturated heterocyclic ring of 5-7 atoms having the formula 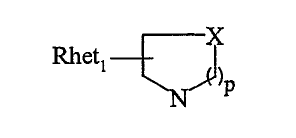 wherein p is 1, 2, 3;
X is selected from CHRhet₁, NRhet₁ and O with the proviso that p is 2 or 3 when X is NRhet₁ and O;
Rhet₁ is selected from hydrogen and C₁₋₅ alkyl, optionally functionalised with OH, halogen (F, Cl and Br), CN, COORhet₂, N(Rhet₂)₂, wherein Rhet₂ independently is selected from H, C₁-₄ alkyl;
with the proviso that when R' and R" are methyl then R cannot be methyl;
and pharmaceutically acceptable salts, hydrates and solvates thereof.

11. Compounds according to claim 10 wherein
R is selected from methyl, ethyl, *n*-propyl and *iso*-propyl,
R_{Na} and R_{Nb} are hydrogen,
one of R₂, R₃, R₅ and R₆ is selected from hydrogen, methyl, methoxy, thiomethyl, hydroxy, fluoro, chloro, trifluoromethyl and phenyl,
with the proviso that when R' and R" are methyl then R cannot be methyl; and
R' and R" are selected from methyl, ethyl, *n*-propyl and *n*-butyl.

12. N-[(3-Diethylamino)propyl]-4-isobutyrylamino-2-methoxy-benzamide according to claims 9 and 10.

13. Compounds of the general formula (I) 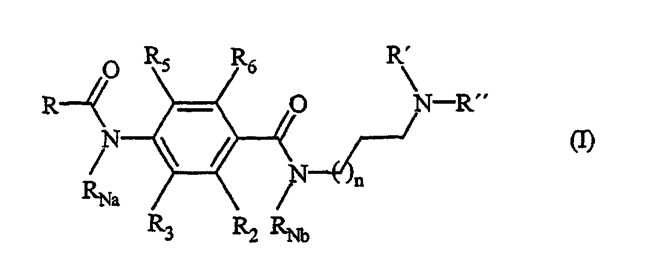 wherein
R is selected from methyl, ethyl, *n*-propyl, *iso*-propyl, *c*-propyl, *n*-butyl, *sec*.-butyl, *iso*-butyl, *tert*.-butyl, *c*-butyl, *n*-pentyl, *sec*.-pentyl, *iso*-pentyl, *tert*.-pentyl, *neo*-pentyl, *c*-pentyl, *c*-hexyl and *c*-heptyl;
R_{Na} and R_{Nb} are the same or different and selected from hydrogen, methyl and ethyl;
R₂, R₃, R₅ and R₆ are independently selected from hydrogen, methyl, methoxy, thiomethyl, hydroxy, fluoro, chloro, bromo, trifluoromethyl, phenyl and benzyl;
n is 1, 2 or 3;
R' and R" are the same or different and selected from methyl, ethyl, *n*-propyl, *iso*-propyl, *n* -butyl, *sec*.-butyl, and *iso*-butyl or R' and R" together form a saturated heterocyclic ring of 5-7 atoms having the formula 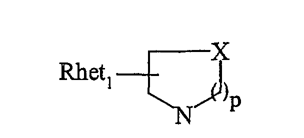 wherein p is 1, 2, 3;
X is selected from CHRhet₁, NRhet₁ and O with the proviso that p is 2 or 3 when X is NRhet₁ and O;
Rhet₁ is selected from hydrogen and C₁₋₅ alkyl, optionally functionalised with OH, halogen (F, Cl and Br), CN, COORhet₂, N(Rhet₂)₂, wherein Rhet₂ independently is selected from H, C₁-₄ alkyl;
with the proviso that when R' and R" are methyl then R cannot be methyl;
and pharmaceutically acceptable salts, hydrates and solvates thereof; for therapeutic use.

## Patentansprüche

1. Verwendung einer Verbindung der allgemeinen Formel (I) 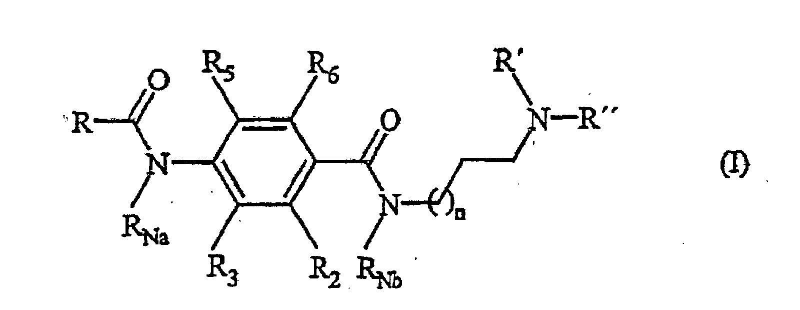 wobei
der Rest R aus Methyl, Ethyl, *n*-Propyl, *iso*-Propyl, *c*-Propyl, *n*-Butyl, *sec*-Butyl, *iso*-Butyl, *tert*-Butyl, *c*-Butyl, *n*-Pentyl, *sec*-Pentyl, *iso*-Pentyl, *tert*-Pentyl, *neo*-Pentyl, *c*-Pentyl, *c*-Hexyl und *c*-Heptyl ausgewählt ist;
die Reste R_{Na} und R_{Nb} gleich oder verschieden und aus Wasserstoff, Methyl und Ethyl ausgewählt sind;
die Reste R₂, R₃, R₅ und R₆ unabhängig aus Wasserstoff, Methyl, Methoxy, Thiomethyl, Hydroxy, Fluor, Chlor, Brom, Trifluormethyl, Phenyl und Benzyl ausgewählt sind;
n gleich 1, 2 oder 3 ist;
die Reste R' und R" gleich oder verschieden und aus Methyl, Ethyl, *n*-Propyl, *iso*-Propyl, *n*-Butyl, *sec*-Butyl und *iso*-Butyl ausgewählt sind oder die Reste R' und R" zusammen einen gesättigten heterocyclischen Ring mit 5 bis 7 Atomen der Formel 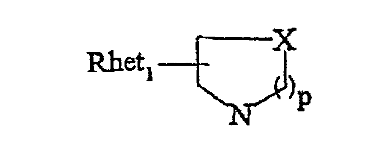 bilden,
wobei p gleich 1, 2, 3 ist;
X aus CHRhet₁, NRhet₁ und O ausgewählt ist, mit der Maßgabe, dass p gleich 2 oder 3 ist, wenn X die Bedeutung NRhet₁ und O hat;
Rhet₁ aus Wasserstoff und C₁₋₅-Alkyl ausgewählt ist, welches gegebenenfalls mit OH, Halogen (F, Cl und Br), CN, COORhet₂, N(Rhet₂)₂ funktionalisiert ist, wobei Rhet₂ unabhängig aus H, C₁₋₄-Alkyl ausgewählt ist; und pharmazeutisch verträglicher Salze, Hydrate und Solvate davon zur Herstellung eines Medikaments zur Stimulierung des Transkriptionsfaktors AP (activator protein)-1.

2. Verwendung gemäß Anspruch 1 der Verbindungen der allgemeinen Formel (I) zur Herstellung eines Medikaments zur Stimulierung, Verbesserung oder Modulation der Immunantwort.

3. Verwendung gemäß Anspruch 2 der Verbindungen der allgemeinen Formel (I) zur Herstellung eines Medikaments zur Behandlung von Krebs und unerwünschter Immunsuppression, welche durch z.B. Zytostatika- und Strahlungstherapie ausgelöst wird.

4. Verwendung gemäß Anspruch 2 der Verbindung der allgemeinen Formel (I) zur Herstellung eines Medikaments zur Behandlung von Autoimmunkrankheit.

5. Verwendung gemäß Anspruch 2 der Verbindungen der allgemeinen Formel (I) zur Herstellung eines Medikaments zur Behandlung von Infektionskrankheit.

6. Verwendung gemäß Anspruch 1 der Verbindungen der allgemeinen Formel (I) zur Herstellung eines Medikaments zur Behandlung von manisch-depressiver Erkrankung.

7. Verwendung gemäß Anspruch 1 von N-[(3-Diethylamino)propyl]-4-isobutyrylamino-2-methoxybenzamid.

8. Verwendung gemäß Anspruch 1 der Verbindung zur Herstellung eines Medikaments, welches in einer täglichen Dosierung zwischen 0,0005 mg/kg bis etwa 10 mg/kg Körpergewicht, insbesondere zwischen 0,005 bis 1 mg/kg Körpergewicht verabreicht werden soll.

9. Zusammensetzung, umfassend als einen Wirkstoff Verbindungen der allgemeinen Formel (I) 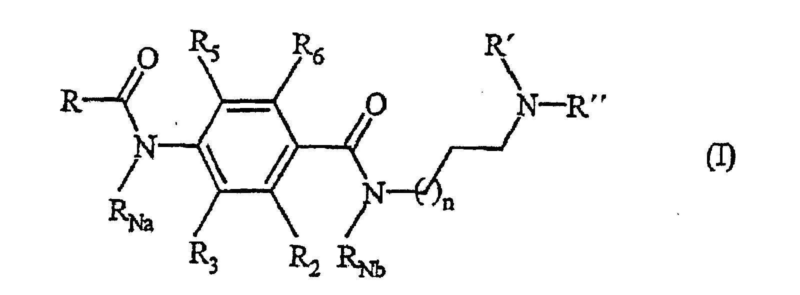 wobei
der Rest R aus Methyl, Ethyl, *n*-Propyl, *iso*-Propyl, *c*-Propyl, *n*-Butyl, *sec*-Butyl, *iso*-Butyl, *tert*-Butyl, *c*-Butyl, *n*-Pentyl, *sec*-Pentyl, *iso*-Pentyl, *tert*-Pentyl, *neo*-Pentyl, *c*-Pentyl, *c*-Hexyl und *c*-Heptyl ausgewählt ist;
die Reste R_{Na} und R_{Nb} gleich oder verschieden und aus Wasserstoff, Methyl und Ethyl ausgewählt sind;
die Reste R₂, R₃, R₅ und R₆ unabhängig aus Wasserstoff, Methyl, Methoxy, Thiomethyl, Hydroxy, Fluor, Chlor, Brom, Trifluormethyl, Phenyl und Benzyl ausgewählt sind;
n gleich 1, 2 oder 3 ist;
die Reste R' und R" gleich oder verschieden und aus Methyl, Ethyl, *n*-Propyl, *iso*-Propyl, *n*-Butyl, *sec*-Butyl und *iso*-Butyl ausgewählt sind oder die Reste R' und R" zusammen einen gesättigten heterocyclischen Ring mit 5 bis 7 Atomen der Formel 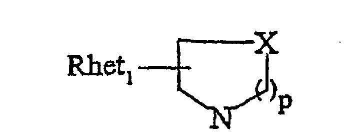 bilden,
wobei p gleich 1, 2, 3 ist;
X aus CHRhet₁, NRhet₁ und O ausgewählt ist, mit der Maßgabe, dass p gleich 2 oder 3 ist wenn X die Bedeutung NRhet₁ und O hat;
Rhet₁ aus Wasserstoff und C₁₋₅-Alkyl ausgewählt ist, welches gegebenenfalls mit OH, Halogen (F, Cl und Br), CN, COORhet₂, N(Rhet₂)₂ funktionalisiert ist, wobei Rhet₂ unabhängig aus H, C₁₋₄-Alkyl ausgewählt ist; pharmazeutisch verträgliche Salze, Hydrate und Solvate davon und einen pharmazeutisch wirksamen Träger.

10. Verbindungen der allgemeinen Formel (I) 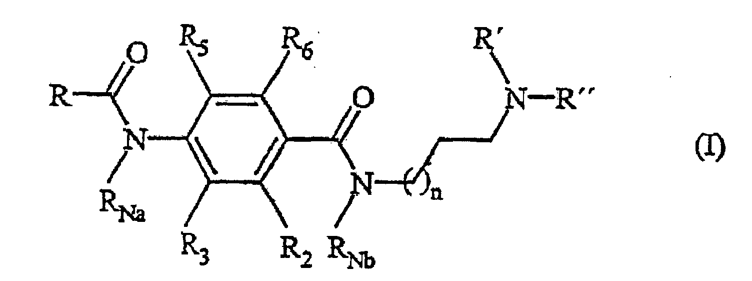 wobei
der Rest R aus Methyl, Ethyl, *n*-Propyl, *iso*-Propyl, *c*-Propyl, *n*-Butyl, *sec*-Butyl, *iso*-Butyl, *tert*-Butyl, *c*-Butyl, *n*-Pentyl, *sec*-Pentyl, *iso*-Pentyl, *tert*-Pentyl, *neo*-Pentyl, *c*-Pentyl, *c*-Hexyl und *c*-Heptyl ausgewählt ist;
die Reste R_{Na} und R_{Nb} gleich oder verschieden und aus Wasserstoff, Methyl und Ethyl ausgewählt sind;
die Reste R₂, R₃, R₅ und R₆ unabhängig aus Wasserstoff, Methyl, Methoxy, Thiomethyl, Hydroxy, Fluor, Chlor, Brom, Trifluormethyl, Phenyl und Benzyl ausgewählt sind;
n gleich 1, 2 oder 3 ist;
die Reste R' und R" gleich oder verschieden und aus Methyl, Ethyl, *n*-Propyl, *iso*-Propyl, *n*-Butyl, *sec*-Butyl und *iso*-Butyl ausgewählt sind oder die Reste R' und R" zusammen einen gesättigten heterocyclischen Ring mit 5 bis 7 Atomen der Formel 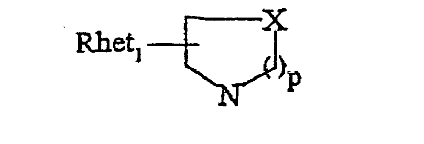 bilden,
wobei p gleich 1, 2, 3 ist;
X aus CHRhet₁, NRhet₁ und O ausgewählt ist, mit der Maßgabe, dass p gleich 2 oder 3 ist, wenn X die Bedeutung NRhet₁ und O hat;
Rhet₁ aus Wasserstoff und C₁₋₅-Alkyl ausgewählt ist, welches gegebenenfalls mit OH, Halogen (F, Cl und Br), CN, COORhet₂, N(Rhet₂)₂ funktionalisiert ist, wobei Rhet₂ unabhängig aus H, C₁₋₄-Alkyl ausgewählt ist;
mit der Maßgabe, dass, wenn die Reste R' und R" Methyl sind, der Rest R nicht Methyl sein kann; und pharmazeutisch verträgliche Salze, Hydrate und Solvate davon.

11. Verbindungen gemäß Anspruch 10, wobei
der Rest R aus Methyl, Ethyl, *n*-Propyl und *iso*-Propyl ausgewählt ist,
die Reste R_{Na} und R_{Nb} Wasserstoff sind,
einer der Reste von R₂, R₃, R₅ und R₆ aus Wasserstoff, Methyl, Methoxy, Thiomethyl, Hydroxy, Fluor, Chlor, Trifluormethyl und Phenyl ausgewählt ist,
mit der Maßgabe, dass, wenn die Reste R' und R" Methyl sind, der Rest R nicht Methyl sein kann; und die Reste R' und R" aus Methyl, Ethyl, *n*-Propyl und *n*-Butyl ausgewählt sind.

12. N-[(3-Diethylamino)propyl]-4-isobutyrylamino-2-methoxybenzamid gemäß den Ansprüchen 9 und 10.

13. Verbindungen der allgemeinen Formel (I) 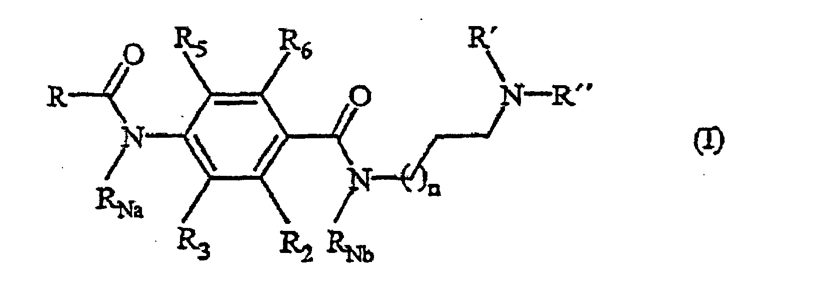 wobei
der Rest R aus Methyl, Ethyl, *n*-Propyl, *iso*-Propyl, *c*-Propyl, *n*-Butyl, *sec*-Butyl, *iso*-Butyl, *tert*-Butyl, *c*-Butyl, *n*-Pentyl, *sec*-Pentyl, *iso-Pentyl, tert*-Pentyl, *neo*-Pentyl, *c*-Pentyl, *c*-Hexyl und *c*-Heptyl ausgewählt ist;
die Reste R_{Na} und R_{Nb} gleich oder verschieden und aus Wasserstoff, Methyl und Ethyl ausgewählt sind;
die Reste R₂, R₃, R₅ und R₆ unabhängig aus Wasserstoff, Methyl, Methoxy, Thiomethyl, Hydroxy, Fluor, Chlor, Brom, Trifluormethyl, Phenyl und Benzyl ausgewählt sind;
n gleich 1, 2 oder 3 ist;
die Reste R' und R" gleich oder verschieden und aus Methyl, Ethyl, *n*-Propyl, *iso*-Propyl, *n*-Butyl, *sec*-Butyl und *iso*-Butyl ausgewählt sind oder die Reste R' und R" zusammen einen gesättigten heterocyclischen Ring mit 5 bis 7 Atomen der Formel 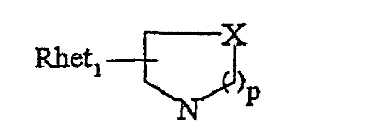 bilden,
wobei p gleich 1, 2, 3 ist;
X aus CHRhet₁, NRhet₁ und O ausgewählt ist, mit der Maßgabe, dass p gleich 2 oder 3 ist, wenn X die Bedeutung NRhet₁ und O hat;
Rhet₁ aus Wasserstoff und C₁₋₅-Alkyl ausgewählt ist, welches gegebenenfalls mit OH Halogen (F, Cl und Br), CN, COORhet₂, N(Rhet₂)₂ funktionalisiert ist, wobei Rhet₂ unabhängig aus H, C₁₋₄-Alkyl ausgewählt ist;
mit der Maßgabe, dass, wenn die Reste R' und R" Methyl sind, der Rest R nicht Methyl sein kann;
und pharmazeutisch verträgliche Salze, Hydrate und Solvate davon zur therapeutischen Verwendung.

## Revendications

1. Utilisation d'un composé de la formule générale (I) 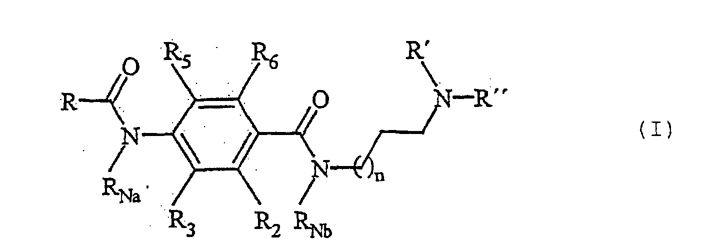 dans laquelle :
R est choisi parmi méthyle, éthyle, n-propyle, iso-propyle, c-propyle, n-butyle, sec-butyle, iso-butyle, tert-butyle, c-butyle, n-pentyle, sec-pentyle, iso-pentyle, tert-pentyle, néo-pentyle, c-pentyle, c-hexyle et c-heptyle ;
R_{Na} et R_{Nb} sont identiques ou différents et sont choisis parmi hydrogène, méthyle et éthyle ;
R₂, R₃, R₅ et R₆ sont choisis indépendamment parmi hydrogène, méthyle, méthoxy, thiométhyle, hydroxy, fluoro, chloro, bromo, trifluorométhyle, phényle et benzyle ;
n est 1, 2 ou 3 ;
R' et R" sont identiques ou différents et sont choisis parmi méthyle, éthyle, n-propyle, iso-propyle, n-butyle, sec-butyle et iso-butyle ou R' et R" forment ensemble un hétérocycle saturé de 5-7 atomes ayant la formule : 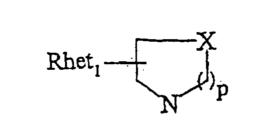
dans laquelle p est 1, 2, 3 ;
X est choisi parmi CHRhet₁, NRhet₁ et O, sous réserve que p est 2 ou 3 lorsque X est NRhet₁ et O ;
Rhet₁ est choisi parmi hydrogène et alkyle en C₁₋₅, éventuellement fonctionnalisé avec OH, halogène (F, Cl et Br), CN, COORhet₂, N(Rhet₂)₂, où Rhet₂ est choisi indépendamment parmi H, alkyle en C₁₋₄ ;
et des sels, des hydrates et des solvates pharmaceutiquement acceptables de ceux-ci ;
pour la préparation d'un médicament pour la stimulation du facteur de transcription AP(protéine activatrice)-1.

2. Utilisation selon la revendication 1 de composés de la formule générale (I) pour la préparation d'un médicament pour la stimulation, l'activation ou la modulation de la réponse immunitaire.

3. Utilisation selon la revendication 2 de composés de la formule générale (I) pour la préparation d'un médicament pour le traitement du cancer et de l'immunosuppression non désirée induite par, par exemple, la thérapie cytostatique et la radiothérapie.

4. Utilisation selon la revendication 2 de composés de la formule générale (I) pour la préparation d'un médicament pour le traitement des maladies auto-immunes.

5. Utilisation selon la revendication 2 de composés de la formule générale (I) pour la préparation d'un médicament pour le traitement des maladies infectieuses.

6. Utilisation selon la revendication 1 de composés de la formule générale (I) pour la préparation d'un médicament pour le traitement de la maladie affective bipolaire.

7. Utilisation selon la revendication 1 de N-[(3-diéthylamino)propyl]-4-isobutyrylamino-2-méthoxybenzamide.

8. Utilisation selon la revendication 1 du composé pour la préparation d'un médicament destiné à être administré dans un dosage quotidien compris entre 0,0005 mg/kg et environ 10 mg/kg de poids corporel, en particulier entre 0,005 et 1 mg/kg de poids corporel.

9. Composition comprenant en tant qu'ingrédient actif des composés de la formule générale (I) 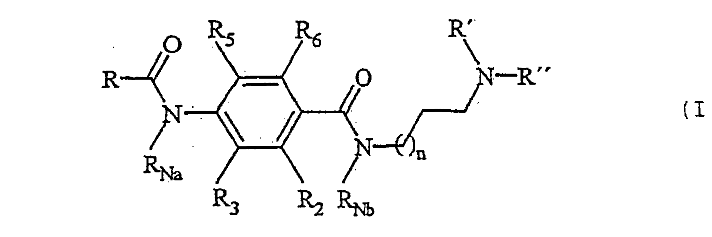 dans laquelle :
R est choisi parmi méthyle, éthyle, n-propyle, iso-propyle, c-propyle, n-butyle, sec-butyle, iso-butyle, tert-butyle, c-butyle, n-pentyle, sec-pentyle, iso-pentyle, tert-pentyle, néo-pentyle, c-pentyle, c-hexyle et c-heptyle ;
R_{Na} et R_{Nb} sont identiques ou différents et sont choisis parmi hydrogène, méthyle et éthyle ;
R₂, R₃, R₅ et R₆ sont choisis indépendamment parmi hydrogène, méthyle, méthoxy, thiométhyle, hydroxy, fluoro, chloro, bromo, trifluorométhyle, phényle et benzyle ;
n est 1, 2 ou 3 ;
R' et R" sont identiques ou différents et sont choisis parmi méthyle, éthyle, n-propyle, iso-propyle, n-butyle, sec-butyle et iso-butyle ou R' et R" forment ensemble un hétérocycle saturé de 5-7 atomes ayant la formule : 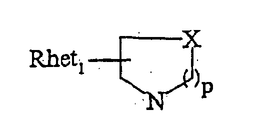
dans laquelle p est 1, 2, 3 ;
X est choisi parmi CHRhet₁, NRhet₁ et O, sous réserve que p est 2 ou 3 lorsque X est NRhet₁ et O ;
Rhet₁ est choisi parmi hydrogène et alkyle en C₁₋₅, éventuellement fonctionnalisé avec OH, halogène (F, Cl et Br), CN, COORhet₂, N(Rhet₂)₂, où Rhet₂ est choisi indépendamment parmi H, alkyle en C₁₋₄ ;
des sels, des hydrates et des solvates pharmaceutiquement acceptables de ceux-ci ;
et un véhicule pharmaceutiquement actif.

10. Composés de la formule générale (I) 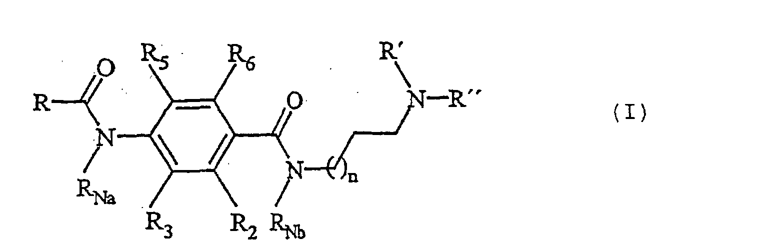 dans laquelle :
R est choisi parmi méthyle, éthyle, n-propyle, iso-propyle, c-propyle, n-butyle, sec-butyle, iso-butyle, tert-butyle, c-butyle, n-pentyle, sec-pentyle, iso-pentyle, tert-pentyle, néo-pentyle, c-pentyle, c-hexyle et c-heptyle ;
R_{Na} et R_{Nb} sont identiques ou différents et sont choisis parmi hydrogène, méthyle et éthyle ;
R₂, R₃, R₅ et R₆ sont choisis indépendamment parmi hydrogène, méthyle, méthoxy, thiométhyle, hydroxy, fluoro, chloro, bromo, trifluorométhyle, phényle et benzyle ;
n est 1, 2 ou 3 ;
R' et R" sont identiques ou différents et sont choisis parmi méthyle, éthyle, n-propyle, iso-propyle, n-butyle, sec-butyle et iso-butyle ou R' et R" forment ensemble un hétérocycle saturé de 5-7 atomes ayant la formule : 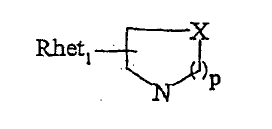
dans laquelle p est 1, 2, 3 ;
X est choisi parmi CHRhet₁, NRhet₁ et O, sous réserve que p est 2 ou 3 lorsque X est NRhet₁ et O ;
Rhet₁ est choisi parmi hydrogène et alkyle en C₁₋₅, éventuellement fonctionnalisé avec OH, halogène (F, Cl et Br), CN, COORhet₂, N(Rhet₂)₂, où Rhet₂ est choisi indépendamment parmi H, alkyle en C₁₋₄ ;
sous réserve que lorsque R' et R" sont un méthyle, alors R ne peut pas être un méthyle ;
et des sels, des hydrates et des solvates pharmaceutiquement acceptables de ceux-ci.

11. Composés selon la revendication 10, dans laquelle :
R est choisi parmi méthyle, éthyle, n-propyle et iso-propyle ;
R_{Na} et R_{Nb} sont un hydrogène ;
l'un de R₂, R₃, R₅ et R₆ est choisi parmi hydrogène, méthyle, méthoxy, thiométhyle, hydroxy, fluoro, chloro, trifluorométhyle et phényle ;
sous réserve que lorsque R' et R" sont un méthyle, alors R ne peut pas être un méthyle ; et
R' et R" sont choisis parmi méthyle, éthyle, n-propyle et n-butyle.

12. N-[(3-Diéthylamino)propyl]-4-isobutyrylamino-2-méthoxy-benzamide selon les revendications 9 et 10.

13. Composés de la formule générale (I) 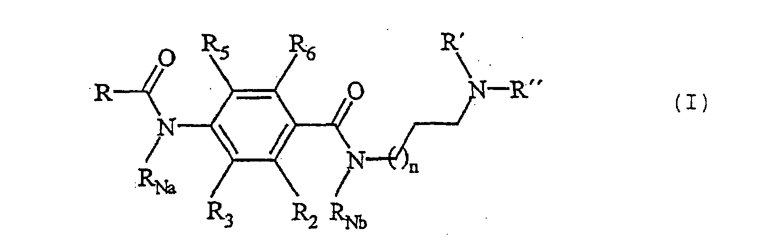 dans laquelle :
R est choisi parmi méthyle, éthyle, n-propyle, iso-propyle, c-propyle, n-butyle, sec-butyle, iso-butyle, tert-butyle, c-butyle, n-pentyle, sec-pentyle, iso-pentyle, tert-pentyle, néo-pentyle, c-pentyle, c-hexyle et c-heptyle ;
R_{Na} et R_{Nb} sont identiques ou différents et sont choisis parmi hydrogène, méthyle et éthyle ;
R₂, R₃, R₅ et R₆ sont choisis indépendamment parmi hydrogène, méthyle, méthoxy, thiométhyle, hydroxy, fluoro, chloro, bromo, trifluorométhyle, phényle et benzyle ;
n est 1, 2 ou 3 ;
R' et R" sont identiques ou différents et sont choisis parmi méthyle, éthyle, n-propyle, iso-propyle, n-butyle, sec-butyle et iso-butyle ou R' et R" forment ensemble un hétérocycle saturé de 5-7 atomes ayant la formule : 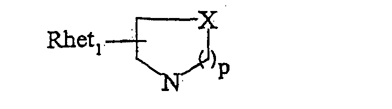
dans laquelle p est 1, 2, 3 ;
X est choisi parmi CHRhet₁, NRhet₁ et O, sous réserve que p est 2 ou 3 lorsque X est NRhet₁ et O ;
Rhet₁ est choisi parmi hydrogène et alkyle en C₁₋₅, éventuellement fonctionnalisé avec OH, halogène (F, Cl et Br), CN, COORhet₂, N(Rhet₂)₂, où Rhet₂ est choisi indépendamment parmi H, alkyle en C₁₋₄ ;
sous réserve que lorsque R' et R" sont un méthyle, alors R ne peut pas être un méthyle ;
et des sels, des hydrates et des solvates pharmaceutiquement acceptables de ceux-ci ;
pour une utilisation thérapeutique.
